Europäisches Patentamt

European Patent Office

Office européen des brevets

(19)

(11) Numéro de publication **0 012 685**
**B1**

(12) FASCICULE DE BREVET EUROPEEN

(45) Date de publication du fascicule du brevet
14.07.82

(51) Int. Cl.³: **B 01 J 31/14,** C 07 C 2/30

(21) Numéro de dépôt: 79400995.1

(22) Date de dépôt: 10.12.79

(54) Nouvelle composition catalytique et sa mise en oeuvre pour l'oligomérisation des oléfines.

(30) Priorité 11.12.78 FR 7835011

(43) Date de publication de la demande:
25.06.80 Bulletin 80/13

(45) Mention de la délivrance du brevet.
14.07.82 Bulletin 82/28

(84) Etats contractants désignés:
BE DE GB IT NL SE

(56) Documents cités:
**GB-A-1 051 564**

(73) Titulaire: **INSTITUT FRANCAIS DU PETROLE, 4, Avenue de Bois-Préau, F-92502 Rueil-Malmaison (FR)**

(72) Inventeur: **Chauvin, Yves, 64, avenue du Général Leclerc, F-78230 Le Pecq (FR)**
Inventeur: **Commereuc, Dominique, 4, Allée des Vignes, F-92190 Meudon (FR)**
Inventeur: **Gaillard, Jean, rue du Commandant Charcot, F-69000 Lyon (FR)**
Inventeur: **Leger, Gérard, Les Grandes Treives 82, Avenue Mérieux, F-69290 Saint Genis Les Ollieres (FR)**
Inventeur: **Phung, Nhu Hung, 1 rue Marie Laure, F-92160 Antony (FR)**

Nouvelle composition catalytique et sa mise en œuvre pour l'oligomérisation des oléfines

L'objet de la présente invention est une nouvelle formule catalytique et sa mise en œuvre pour l'oligomérisation, en particulier la dimérisation et la trimérisation, des monooléfines. Elle concerne plus précisément certaines combinaisons obtenues par mise en contact, dans un ordre quelconque, d'au moins un composé de nickel bivalent, avex au moins un halogénure d'hydrocarbylaluminium et au moins un acide organique de Bronsted.

Il est déjà connu de préparer des catalyseurs de dimérisation ou de codimérisation de monooléfines telles que l'éthylène, le propylène ou les n-butènes. Parmi ces catalyseurs ont été notamment décrits les produits d'interaction des halogénures de $\pi$-allyl nickel phosphine avec les acides de Lewis (brevet français 1.410.430) et les produits d'interaction des halogénures de nickel phosphine avec les acides de Lewis (brevet des Etats-Unis 3.485.881), les catalyseurs utilisent un ligand consistant en un composé organique du phosphore. Or il est souhaitable de pouvoir disposer de catalyseurs d'oligomérisation sans phosphore. On connait de tels catalyseurs, notamment les produits d'interaction de certains carboxylates de nickel avec des halogénures d'hydrocarbylaluminium (brevet des Etats-Unis 3.321.546 ou brevet britannique 1.051.564). D'autres catalyseurs mettent en œuvre des composés zérovalents du nickel qui sont d'un emploi peu pratique en raison de leur instabilité et de leur coût.

On a proposé aussi d'utiliser des catalyseurs dans lesquels le nickel se trouve déposé sur un support minéral à sites acides, par exemple silice, silicealumine, phosphate d'aluminium. Il s'agit là de catalyseurs à phase solide, contrairement aux catalyseurs à phase liquide de l'invention. Leur activité est per ailleurs faible.

Enfin, une autre difficulté de la technique antérieure est la suivante: la mise en œuvre industrielle en continu des compositions catalytiques connues sur des coupes oléfinique telles qu'elles sont issues des procédés pétrochimiques comme le cracking catalytique ou le cracking à la vapeur, se heurte à des difficultés liées d'une part aux impuretés contenues dans ces coupes et d'autre part au fait que l'on constate une activité souvent plus faible qu'en système fermé dans lequel on introduit tous les constituants une fois pour toutes en début de réaction, et que cette activité va en décroissant au cours du temps.

Il a été trouvé de façon inattendue que l'association d'un composé du nickel bivalent, d'un halogénure d'hydrocarbyle aluminium et d'un composé à caractère acide de Bronsted ne renfermant pas de phosphore conduisait à une composition catalytique qui, en particulier dans un procédé en continu, est plus active que les précédentes, conserve une plus grande stabilité dans le temps et est moins sensible aux impuretés qui se trouvent à l'état de traces dans les charges oléfiniques.

Comme composé du nickel on peut utiliser tous les compsés du nickel bivalent, de préférence solubles à plus de 1 g. par litre en milieu hydrocarboné (exemple: dans le n-heptane à 20° C) et plus particulièrement dans les réactifs ou le milieu de réaction, de préférence les carboxylates de formule générale $(RCOO)_2Ni$ où R est un reste hydrocarbyl, par exemple alkyl, cycloalkyl, alkényl, aryl, aralkyl ou alkaryl contenant jusqu'à 20 atomes de carbone, de préférence un reste hydrocarbyl de 5 à 20 atomes de carbone. Les deux radicaux R peuvent aussi constituer un reste alkylène de 6 à 18 atomes de carbone. Des exemples de composés du nickel sont les sels de nickel bivalent suivants: octoate, éthyl-2 hexanoate, décanoate, stéarate, oléate, salicylate, acétylacétonate, hydroxydécanoate. De nombreux autres exemples peuvent être trouvés dans la littérature et les brevets, et l'invention n'est pas limitée aux seuls exemples donnés plus haut. Le radical R peut être substitué par 1 à 4 (ou davantage) atomes d'halogène, groupes hydroxy, cétone, nitro, cyano ou autres groupes qui ne gènent pas la réaction.

Les composés d'halogénures d'hydrocarbylaluminium répondent à la formule générale $AlR_xX_y$ où R représente un groupement hydrocarboné contenant par exemple jusqu'a 12 atomes de carbone tel que alkyl, aryl, aralkyl, alkaryl, cycloalkyl; X représente le chlore ou le brome et x a une valeur de 1 à 1,5, y une valeur de 1,5 à 2, de préférence x = 1 et y = 2, avec x + y = 3. Comme exemples de tels composés on peut mentionner le sesquichlorure d'éthylaluminium, le dichloroéthylaluminium, le dichloroisobutylaluminium et le dibrométhylaluminium.

L'acide de Bronsted est un composé de formule HX où X est un anion organique ne renfecment pas de phosphore, par exemple corboxylique, sulfonique ou phénolique. On utilise les acides de $pK_a$ à 20° C au maximum égal à 3, plus particulièrement ceux qui sont solubles dans le composé du nickel ou dans sa solution dans un hydrocarbure ou autre solvant convenable, à la concentration désirée, et qui ne renferment pas de phosphore. Une classe d'acides ã laquelle il faut donner la préférence inclut les acides halogénocarboxyliques de formule $R_1COOH$ dans laquelle $R_1$ est un radical alkyl halogéné, notamment ceux qui renferment au moins un atome d'halogène en $\alpha$ du groupe COOH avec, au total, de 2 à 10 atomes de carbone. Une classe d'acides à laquelle il faut donner la préférence inclut les acides halogénocarboxyliques de formule $R_1COOH$ dans laquelle $R_1$ est un reste halogénoalkyl renfermant de 1 à 3 atomes de carbone, de formule $C_mH_pX_q$ où X est un halogène (fluor, chlore, brome ou iode), m = 1, 2 ou 3, p est égal à zéro ou à un nombre entier et q est un nombre entier, avec la condition que $p + q = 2m + 1$. On utilise le plus aventageusement un acide halogénoacétique de formule $R_2COOH$ dans laquelle $R_2$ est un reste halogénométhyle de formule $CX_nH_{3-n}$ où X est le fluor, le

2

chlore, le brome ou l'iode, avec n entier de 1 à 3. On peut citer comme acides utilisables, les acides trifluoroacétique, difluoroacétique, monofluoroacétique, trichloroacétique, dichloroacétique, mono-chloroacétique, tribromoacétique, dibromoacétique, monobromoacétique, triiodoacétique, diiodoacé-tique, monoiodeacétique, pentafluoropropionique, fluoro-2-propionique, dichloro-2,2 propionique, chloro-2-propionique, heptafluorobutyrique, fluoro-2-butyrique ou chloro-2-butyrique. Les exemples précédents ne sont pas limitatifs. D'autres acides utilisables sont, par exemple, les acides arylsulfoniques, alkylsulfoniques, l'acide picrique, l'acide nitroacétique, les acides dinitrobenzoïques, l'acide o-nitrobenzoïque et l'acide cyanacétique.

L'invention a aussi pour objet un procédé d'oligomérisation de monooléfines en présence du système catalytique ci-dessus défini à une température de $-20°C$ à $+60°C$ dans des conditions de pression telle que les réactifs soient maintenus au moins en majorité en phase liquide ou en phase condensée.

Les monooléfines susceptibles d'être dimérisées ou oligomérisées sont, par exemple, l'éthylène, le propylène, les n-butènes, les n-pentènes pus ou sous forme de mélanges tels qu'issus des procédés de synthèse comme le cracking catalytique. Elles peuvent être cooligomérisées entre elles ou avec l'isobutène, par exemple l'éthylène avec le propylène et les n-butènes, le propylène avec les n-butènes, les n-butènes avec l'isobutène.

La concentration, exprimée en nickel, de la composition catalytique dans la phase liquide de réaction d'oligomérisation est normalement comprise entre 5 et 500 parties par million en poids. Le rapport molaire de l'halogénure d'hydrocarbylaluminium au composé du nickel est normalement compris entre 1 : 1 et 50 : 1 et plus avantageusement compris entre 2 : 1 et 20 : 1. Le rapport molaire de l'acide de Bronsted au composé de l'aluminium est aventageusement compris entre 0,001 : 1 et 1 : 1, de préférence entre 0,01 : 1 et 0,5 : 1; la valeur préférée du rapport molaire de l'acide de Bronsted au composé du nickel est de 0,25 : 1 à 5 : 1.

Le procédé peut être mis en œuvre dans un réacteur à un ou plusieurs étages de réaction en série, la charge oléfinique et/ou les constituants du système catalytique étant introduits en continu, soit dans le premier étage soit dans le premier et un quelconque des étages. On peut également n'introduire dans le second et/ou le »n« ième étage qu'un ou deux des constituants du mélange catalytique.

A la sortie du réacteur, le catalyseur peut être désactivé, par exemple à l aide d'ammoniac et/ou d'une solution aqueuse de soude et/ou d'une solution aqueuse d'acide sulfurique. Les oléfines non converties et les alcanes sont ensuite séparés des oligomères par distillation.

Les produits du présent procédé trouvent leur application comme composant d'un carburant pour voiture automobile ou comme charge d'un procédé d'hydroformylation pour la synthèse d'aldéhydes et d'alcools.

Les exemples suivants sont donnés à titre illustratif et n'entendent en rien limiter l'invention.


Exemple comparatif n° 1

Cet exemple ne fait pas partie de l'invention et n'est destiné qu'à en montrer les aventages.

Le réacteur d'oligomérisation est constitué d'un récipient cylindrique en acier, couplé à un système de recirculation extérieur d'un débit de 2 m³/h destiné à assurer l'homogénéité du mélange réactionnel et à un échangeur de chaleur. Le volume réactionnel total est de 7,2 litres. On introduit en continu dans le système de recirculation: 1 kg/h d'une coupe $C_3$ issue d'un cracking catalytique et contenant approximativement 70% en poids de propylène et 30% en poids de propane; 0,33 g/h de dichloroéthylaluminium sous la forme d'une solution dans l'isooctane; 0,1 g/h d'un carboxylate $C_9-C_{13}$ de nickel à 10% en poids de métal, sous la forme d'une solution dans l'isooctane. On maintient la pression du réacteur à 15 bars par soutirage en continu du produit de réaction, et la température à 42°C par contrôle du débit de l'échangeur.

Après 3 jours de marche, la conversion du propylène en un mélange constitué principalement de dimères et de trimères, se stabilise à 70%. Compte-tenu du propylène récupéré, le rendement en dimères et trimères du propylène est de 97%.


Exemple n° 2

Dans le même appareillage que celui de l'exemple n° 1, et dans les même conditions opératoires, on a introduit, aux mêmes débits, la coupe $C_3$ et les constituants du catalyseur à ceci près que la solution de carboxylate de nickel contenait de l'acide trifluoroacétique à une concentration telle qu'il était introduit au débit de 0,02 g/h. Après 3 jours de marche la conversion du propylène en un mélange d'oligomères sensiblement identique à celui de l'exemple précédent, se stabilisait à 91%. Rendement: 97% comme dans l'exemple 1.

## Exemple comparatif n° 3

Le réacteur d'oligomérisation est constitué de 2 étages de réaction montés en série et composés, chacun, d'un réacteur cylindrique en acier couplé à un système de recirculation externe et à un échangeur de chaleur. Le volume de chaque étage est de 7,2 litres.

On introduit en continu, dans le système de recirculation du 1er étage: 1 kg/h d'une coupe $C_4$ dont la composition est la suivante:

| | |
|---|---|
| butane et isobutane | 23,5% |
| isobutène | 12,0% |
| butène-1 | 22,9% |
| butène-2 trans | 23,2% |
| butène-2 cis | 18,4% |

2,8 g/h de dichloroéthylaluminium en solution dans l'isohexane; 0,78 g/h d'un carboxylate de nickel à 10% en poids de métal, sous la forme d'une solution dans l'isohexane. On maintient la pression du réacteur à 5 bars par soutirage en continu du produit de réaction, et la température à 42°C par contrôle du débit d'eau de l'échangeur.

Une partie des n-butènes et de l'isobutène sont convertis en oligomères composés essentiellement de dimères, codimères, trimères et cotrimères ainsi que des polymères.

Après trois jours de marche, les conversions se sont stabilisées dans le 1er étage à 45% pour les n-butènes et à 80% pour l'isobutène; dans le 2ème étage à 67% pour les n-butènes et à 90% pour l'isobutène. On retrouve 95% des n-butènes sous forme de dimères, trimères, codimères et cotrimères.

## Exemple n° 4

Dans le même appareillage que celui de l'exemple 3 et dans les mêmes conditions opératoires, on a introduit, aux même débits, la coupe $C_4$ et les constituants du catalyseur à ceci près que la solution de carboxylate de nickel contenait de l'acide trifluoroacétique à une concentration telle qu'il était introduit au débit de 0,15 g/h. Après 3 jours de fonctionnement la conversion des butènes en un même mélange d'oligomères, se stabilisait comme suit: dans le 1er étage 67% pour les n-butènes et 88% pour l'isobutène; dans le 2ème étage 76% pour les n-butènes et 98% pour l'isobutène. On retrouve 95% des n-butènes sous forme de dimères, trimères, codimères et cotrimères.

## Exemple n° 5

Le réacteur d'oligomérisation est constitué d'un récipient cylindrique en acier, couplé à un système de recirculation extérieur d'un débit de 770 l/h et à un échangeur de chaleur. Le volume réactionnel total est de 35 litres. On introduit en continu dans le système de recirculation 5 kg/h d'une coupe $C_3$ contenant approximativement 70% en poids de propylène, et 30% en poids de propane; 1,65 g/h de dichloroéthylaluminium sous la forme d'une solution dans l'isooctane; 0,30 g/h d'éthyl-2-hexanoate de nickel, sous la forme d'une solution dans l'isooctane, qui contient en outre de l'acide trichloroacétique à une concentration telle qu'il est introduit au débit de 0,14 g/h. On maintient la pression du réacteur à 15 bars par soutirage en continu du produit de réaction, et la température à 42°C par contrôle du débit de l'échangeur.

Ces conditions de marche correspondent à un temps de séjour moyen et à une concentration en composés de nickel et d'aluminium identiques à ceux de l'exemple n° 1.

Après 2 jours de marche, la conversion du propylène en un mélange constitué principalement de dimères et de trimères se stabilise à 90%. Rendement: 97%.

## Exemples n° 6 à 12

Dans le même appareillage que celui de l'exemple n° 1 et dans les mêmes conditions opératoires, on a introduit, aux mêmes débits, la coupe $C_3$ et les constituants du catalyseur à ceci près que la solution de carboxylate de nickel contenait un acide de Bronsted tel qu'indiqué dans le tableau n° 1, à une concentration telle que le rapport molaire de l'acide de Bronsted au composé de nickel était égal à 1, sauf indication contraire. La conversion du propylène est donnée dans le tableau n° 1. Le rendement, dans tous les cas, était d'environ 97%.

Tableau 1

| Exemple n" | Acide de Bronsted | Conversion du propylène (%) |
|---|---|---|
| 6 | $CH_2F$ COOH | 83 |
| 7 | CH $Cl_2$COOH | 86 |
| 8*) | $CH_2Cl$ COOH | 82 |
| 9 | $CBr_3$ COOH | 85 |
| 10 | $CH_2$ Br COOH | 80 |
| 11 | $CH_2$ I COOH | 78 |
| 12 | $C_3F_7 \cdot$ COOH | 89 |

*) Dans l'exemple 8, le composé d'aluminium était le dichloroisobutylaluminium, le rapport molaire Al/Ni était de 10 : 1 et le rapport molaire acide de Bronsted/composé de nickel était égal à 2.


Exemple n° 13

On introduit 58 g/h d'un mélange liquide à 11,6% en poids d'éthane et 88,4% en poids d'éthylène dans un réacteur sous une pression de 15 bars. Le catalyseur est introduit à raison de 0,046 g/h de Al Et $Cl_2$, 0,013 g/h d'alcane carboxylate $C_8-C_{10}$ de nickel et de l'acide trifluoracétique en rapport molaire de 1 : 1 par rapport au composé du nickel. La température est maintenue à 45° C.

98% de l'éthylène est converti. On obtient un rendement de 98% en poids d'un produit dont l'analyse est la suivants (en moles):

| | |
|---|---|
| $C_4$: | 46,4% |
| $C_6$: | 29,1% (mélange d'hexènes et de méthylpentènes) |
| $C_8$: | 12,0% |
| $C_{10}$: | 5,9% |
| $C_{12}-C_{16}$: | 6,6%. |


**Revendications**

1. Composition catalytique caractérisée en ce qu'elle est le produit résultant de la mise en contact, dans un ordre quelconque, d'au moins un composé de nickel bivalent, avec au moins un halogénure d'hydrocarbylaluminium de formule Al $R_x X_y$ où R est un groupe hydrocarbone monovalent, X est le chlore ou le brome, x a une valeur de 1 à 1,5 et y une valeur de 1,5 à 2 avec x + y = 3, et au moins un acide organique de Bronsted ne renfermant pas de phosphore et dont le $pK_a$ à 20° C est au plus égal à 3.

2. Composition catalytique selon la revendication 1, caractérisée en ce que l'acide de Bronsted est un acide halogénocarboxylique de formule $R_1$ COOH dans laquelle $R_1$ est un reste halogénoalkyl renfermant de 1 à 3 atomes de carbone, de formule $C_m H_p X_q$ où X est un halogène, m = 1, 2 ou 3, p est égal à zéro ou à un nombre entier et q est un nombre entier, avec la condition que p + q = 2m + 1.

3. Composition catalytique selon la revendication 2, caractérisée en ce que l'acide de Bronsted est un acide halogénoacétique de formule $R_2$COOH dans laquelle $R_2$ est un reste halogénométhyle de formule $CX_nH_{3-n}$, où X est un halogène et n est un nombre entier de 1 à 3.

4. Composition catalytique selon la revendication 3, caractérisée en ce que l'acide de Bronsted est l'acide trifluoroacétique, trichloracétique ou tribromoacétique.

5. Composition selon l'une quelconque des revendications 1 à 4, caractérisée en ce que, dans la formule de l'halogénure d'hydrocarbylaluminium, x est égal à 1 et y à 2 et X est le chlore.

6. Composition catalytique selon une quelconque des revendications 1 à 5, caractérisée en ce que le composé de nickel est un carboxylate de nickel de formule $(RCOO)_2Ni$ où chaque R est un reste hydrocarbyl de 5 à 20 atomes de carbone ou les deux R forment ensemble un radical alkylène de 6 à 18 atomes de carbone.

5

7. Composition catalytique selon une quelconque des revendications 1 à 6, dans laquelle le rapport molaire de l'halogénure d'hydrocarbylaluminium au composé de nickel est de 1 : 1 à 50 : 1 et le rapport molaire de l'acide de Bronsted à l'halogénure d'hydrocarbylaluminium est de 0,001 : 1 à 1 : 1.

8. Composition catalytique selon une quelconque des revendications 1 à 6, dans laquelle le rapport molaire de l'halogénure d'hydrocarbylaluminium au composé de nickel est de 2 : 1 à 20 : 1, le rapport molaire de l'acide de Bronsted à l'halogénure d'hydrocarbylaluminium est de 0,01 : 1 à 0,5 : 1 et le rapport molaire de l'acide de Bronsted au composé de nickel est de 0,25 : 1 à 5 : 1.

9. Composition selon l'une des revendications 1 à 8, dans laquelle le composé de nickel présente une solubilité d'au moins 1 g/litre dans le n-heptane à 20° C.

10. Procédé de conversion d'au moins une monooléfine en dimères, trimères et/ou oligomères, dans lequel ladite monooléfine est mise en contact avec une composition catalytique selon l'une quelconque des revendications 1 à 9 à une température de $-20°$ C à $+60°$ C et et sous une pression suffisante pour maintenir les réactifs au moins en majorité en phase liquide ou en phase condensée.

## Patentansprüche

1. Katalytische Komposition, dadurch gekennzeichnet, daß man sie herstellt, indem man in beliebiger Reihenfolge mindestens eine zweiwertige Nickelverbindung mit mindestens einem Hydrocarbyl-Aluminiumhalogenid der Formel $AlR_xX_y$, in der R einen einwertigen Kohlenwasserstoffrest, X Chlor oder Brom bedeutet und x einen Wert von 1 bis 1,5 und y einen Wert von 1,5 bis 2 hat, wobei $x + y = 3$ ist, sowie mit mindestens einer organischen Brönsted-Säure in Kontakt bringt, die keinen Phosphor enthält und deren $pK_a$-Wert bei 20° C höchstens 3 beträgt.

2. Katalytische Komposition gemäß Anspruch 1, dadurch gekennzeichnet, daß die Brönsted-Säure eine Halogen-Carbonsäure der Formel R COOH ist, in welcher $R_1$ einen Halogenalkyl-Rest mit 1 bis 3 Kohlenstoffatomen der Formel $C_mH_pX_q$ bedeutet, in der X ein Halogenatom, $m = 1,2$ oder 3, p gleich 0 oder eine ganze Zahl und q eine ganze Zahl ist, unter der Voraussetzung, daß $p + q = 2m + 1$.

3. Katalytische Komposition gemäß Anspruch 2, dadurch gekennzeichnet, daß die Brönsted-Säure eine Halogen-Essigsäure der Formel $R_2COOH$ ist, in welcher $R_2$ ein Halogenmethyl-Rest der Formel $CX_nH_{3\,n}$ ist, in welcher X ein Halogenatom und n eine ganze Zahl von 1 bis 3 bedeutet.

4. Katalytische Komposition gemäß Anspruch 3, dadurch gekennzeichnet, daß die Brönsted-Säure Trifluoressigsäure, Trichloressigsäure oder Tribromessigsäure ist.

5. Katalytische Komposition gemäß Ansprüchen 1 bis 4, dadurch gekennzeichnet, daß in der Formel des Hydrocarbyl-Aluminiumhalogenids $x = 1$ und $y = 2$ sowie X Chlor ist.

6. Katalytische Komposition gemäß Ansprüchen 1 bis 5, dadurch gekennzeichnet, daß die Nickelverbindung ein Nickelcarboxylat der Formel $(RCOO)_2Ni$ ist, in welcher jedes R ein Hydrocarbyl-Rest mit 5 bis 20 Kohlenstoffatomen ist oder die beiden Reste R zusammen einen Alkylen-Rest mit 6 bis 18 Kohlenstoffatomen bilden.

7. Katalytische Komposition gemäß Ansprüchen 1 bis 6, dadurch gekennzeichnet, daß das Molverhältnis von Hydrocarbyl-Aluminiumhalogenid zur Nickelverbindung 1 : 1 bis 50 : 1 und das Molverhältnis von Brönsted-Säure zum Hydrocarbyl-Aluminiumhalogenid 0,001 : 1 bis 1 : 1 ist.

8. Katalytische Komposition gemäß Ansprüchen 1 bis 6, dadurch gekennzeichnet, daß das Molverhältnis von Hydrocarbyl-Aluminiumhalogenid zur Nickelverbindung 2 : 1 bis 20 : 1, das Molverhältnis von Brönsted-Säure zu Hydrocarbyl-Aluminiumhalogenid 0,01 : 1 bis 0,5 : 1 und das Molverhältnis von Brönsted-Säure zur Nickelverbindung 0,25 : 1 bis 5 : 1 ist.

9. Katalytische Komposition gemäß Ansprüchen 1 bis 8, dadurch gekennzeichnet, daß die Nickelverbindung eine Löslichkeit von mindestens 1 g/Liter in n-Heptan bei 20° C hat.

10. Verfahren zur Umwandlung mindestens eines Monoolefins zu den Dimeren, Trimeren und/oder Oligomeren, dadurch gekennzeichnet, daß das Monoolefin mit einer katalytischen Komposition gemäß Ansprüchen 1 bis 9 bei einer Temperatur von $-20°$ bis $+60°$ C und unter einem ausreichenden Druck in Kontakt gebracht wird. um die Reaktionskomponenten mindestens größtenteils in flüssiger oder kondensierter Phase zu halten.

## Claims

1. A catalyst composition characterized as being the product obtained by contacting, in any order, at least one bivalent nickel compound with at least one hydrocarbylaluminium halide of the formula $AlR_xX_y$ wherein R is a monovalent hydrocarbon group, X is chlorine or bromine, x has a value from 1 to 1.5 and y a value from 1.5 to 2, with $x + y = 3$, and at least one phosphorus-free organic Bronsted acid whose $pK_a$ at 20° C is at most equal to 3.

2. A catalyst composition according to claim 1, wherein the Bronsted acid is a halogenocarboxylic acid of the formula $R_1COOH$ wherein $R_1$ is a halogenalkyl radical containing from 1 to 3 carbon atoms, complying with the formula $C_mH_pX_q$ wherein X is halogen, $m = 1, 2$ or 3, p is zero or an integer and q is an integer, with the condition that $p + q = 2m + 1$.

3. A catalyst composition according to claim 2, wherein the Bronsted acid is a halogenoacetic acid of the formula $R_2COOH$ where $R_2$ is a halogenomethyl group of the formula $CX_nH_{3-n}$, X being halogen and n an integer from 1 to 3.

4. A catalyst composition according to claim 3, wherein the Bronsted acid is trifluoroacetic, trichloracetic or tribromoacetic acid.

5. A composition according to any of claims 1 to 4, wherein, in the formula of the hydrocarbylaluminium halide, x is 1, y is 2 and X is chlorine.

6. A catalyst composition according to any of the claims 1 to 5, wherein the nickel compound is a nickel carboxylate of the formula $(RCOO)_2Ni$ where each R is a hydrocarbyl radical having from 4 to 20 carbon atoms or where the two R form together an alkylene radical having from 6 to 18 carbon atoms.

7. A catalyst composition according to any of the claims 1 to 6, wherein the molar ratio of the hydrocarbylaluminium halide to the nickel compound is from 1 : 1 to 50 : 1 and the molar ratio of the Bronsted acid to the hydrocarbylaluminium halide is from 0.001 : 1 to 1 : 1.

8. A catalyst composition according to any of the claims 1 to 6, wherein the molar ratio of the hydrocarbylaluminium halide to the nickel compound is from 2 : 1 to 20 : 1, the molar ratio of the Bronsted acid to the hydrocarbylaluminium halide is from 0.01 : 1 to 0.5 : 1 and the molar ratio of the Bronsted acid to the nickel compound is from 0.25 : 1 to 5 : 1.

9. A composition according to one of the claims 1 to 8, wherein the nickel compound has a solubility of at least 1 g/liter in n-heptane at 20°C.

10. A process for converting at least one monoolefin to dimers, trimers and/or oligomers, wherein said monoolefin is contacted with a catalyst composition according to any of the claims 1 to 9 at a temperature from −20°C to +60°C and under sufficient pressure to maintain the reactants at least in major part in liquid phase or in condensed phase.